# EUROPÄISCHE PATENTSCHRIFT

(11) **EP 2 601 255 B1**
(45) Veröffentlichungstag und Bekanntmachung des Hinweises auf die Patenterteilung: **27.07.2016**
(21) Anmeldenummer: 11746465.1
(22) Anmeldetag: 01.08.2011
(51) Int. Cl.: C08K 5/00, C08K 5/101

(54) **GLEITMITTELKOMBINATION FÜR DIE VERARBEITUNG VON THERMOPLASTISCHEN KUNSTSTOFFEN**
LUBRICANT COMBINATION FOR THE PROCESSING OF THERMOPLASTIC RESINS
COMBINAISON DE LUBRIFIANTS POUR LE TRAITEMENT DE MATIÈRES THERMOPLASTIQUES

(30) Priorität: 02.08.2010 DE 102010033035
(43) Veröffentlichungstag der Anmeldung: 12.06.2013
(73) Patentinhaber: Emery Oleochemicals GmbH, 40599 Düsseldorf (DE)
(72) Erfinder: DAUTE, Peter, 27616 Beverstedt (DE); SCHWEICHLER, Thomas, 27612 Loxstedt (DE)
(74) Vertreter: Kinkeldey, Daniela
(86) Internationale Anmeldenummer: PCT/EP2011/003846
(87) Internationale Veröffentlichungsnummer: WO 2012/016674

(56) Entgegenhaltungen:
- EP-A1- 1 626 064
- WO-A1-2005/082991
- WO-A1-2011/072346
- US-A- 2 902 500

## Beschreibung

Die vorliegende Erfindung betrifft eine thermoplastische Zusammensetzung, ein Verfahren zur Herstellung einer thermoplastischen Zusammensetzung, die durch dieses Verfahren erhältliche thermoplastische Zusammensetzung, ein Verfahren zur Herstellung eines auf einer thermoplastischen Zusammensetzung basierenden Formkörpers, einen durch dieses Verfahren erhältlichen Formkörper sowie die Verwendung einer Reaktionsmischung.

Thermoplastische Polymere erfreuen sich großer Beliebtheit bei der Herstellung von Formkörpern aller Art. Üblicherweise erfolgt die Verarbeitung thermoplastischer Polymerer zu solchen Formkörpern durch mehrere Schritte, wobei in der Regel mindestens ein Schritt ein Erwärmen des Polymeren umfasst, wobei das Erwärmen häufig bis zu einer Temperatur durchgeführt wird, bei der das thermoplastische Polymere ein für die weitere Verarbeitung ausreichendes rheologisches Profil aufweist. Weitere Verarbeitungsschritte sind oft mit einer intensiven Durchmischung von thermoplastischen Polymeren und weiteren Inhaltsstoffen und mit einer Scherung des Polymeren verbunden. Im Rahmen solcher weiteren Verarbeitungsschritte wird beispielsweise durch Extrusion eine innige Durchmischung der unterschiedlichen Bestandteile einer den Formkörper ergebenden Mischung eines thermoplastischen Polymeren und weiterer Inhaltsstoffe erreicht.

Zur Verbesserung der Verarbeitbarkeit zu Formkörpern wird solchen Mischungen aus thermoplastischen Polymeren und ggf. weiteren Inhaltsstoffen häufig ein sogenanntes Gleitmittel zugefügt. Bei einem solchen Gleitmittel handelt es sich in der Regel um eine niedermolekulare Verbindung aus der Gruppe der Fettsäuren, Fettsäureester, Wachsester, Fettalkoholester, Amidwachse, Metallseifen, Montansäuren, Montansäureester oder um hochmolekulare, polymere Verbindungen, wie Paraffine oder Polyethylenwachse. Insbesondere Polyvinylchlorid lässt sich ohne den Zusatz von Gleitmitteln thermoplastisch nicht verarbeiten. Dies gilt vor allem für die thermoplastische Verarbeitung von Hart-PVC, also von PVC-Massen, welche keine oder nur sehr geringe Weichmacheranteile haben. Beim Einsatz von Gleitmitteln unterscheidet man zwischen sogenannten *"externen Gleitmitteln"* und *"internen Gleitmitteln".* Einen Überblick über die Anwendung von Gleitmitteln gibt Worschech, K. in: Becker, G.; Braun, D.; (Hrsg.) Kunststoff-Handbuch, 2. Aufl., Bd. 2/1: Polyvinylchlorid (1986), S. 570ff.

Gleitmittel, welche als Trennmittel zwischen der Kunststoffmasse und dem Metall des Verarbeitungswerkzeuges wirken, werden als *"externe Gleitmittel"* bezeichnet. Das Gleitmittel soll dabei verhindern, dass der plastifizierte Kunststoff während der Verarbeitung am heißen Metall der Verforrnungswerkzeuge, beispielsweise Förderschnecken von Extrudern oder Metallwalzen von Kalandern, festklebt. Beispiele für extern wirkende Gleitmittel (auch *"Formtrennmittel"* genannt) sind Montansäurewachsester, wie sie etwa in der DE-A 36 43 968 oder der EP-A 346717 beschrieben werden, Fettsäurekomplexester aus Polyolen, Dicarbonsäuren und Fettsäuren, Metallseifen wie Schwermetallstearate, Paraffinwachse oder Paraffinöle, Polyethylenwachse oder Amidwachse wie Ethylendiamindistearat.

Als *"interne Gleitmittel"* werden solche Gleitmittel bezeichnet, welche für eine verbesserte Fließfähigkeit der Schmelze sorgen. Beispiele für intern wirkende Gleitmittel sind etwa Ester der Stearinsäure wie Butylstearat, Fettsäurepartialester des Glycerins wie Glycerinmonooleat oder Phthalsäureester von Fettalkoholen wie Distearylphthalat.

Aus ökologischen und ökonomischen Gründen wäre die direkte Verwendung von gesättigten Triglyceriden wie gehärteter Talg oder gehärtetes Rüböl als Gleitmittel besonders vorteilhaft. Diese Verbindungen wirken jedoch sehr extern (s. Becker/Braun, Kunststoff Handbuch Band 2/1, Carl Hanser Verlag 1986, S. 570-595) und können so nicht eingesetzt werden. Der Einsatz von Abmischungen von gesättigten Triglyceriden mit anderen Kunststoffadditven wird in der WO-A-2005/082991 beschrieben.

Der vorliegenden Erfindung lag die Aufgabe zugrunde, die sich aus dem Stand ergebenen Nachteile im Zusammenhang mit thermoplastischen Formmassen zumindest teilweise zu überwinden.

Insbesondere lag der vorliegenden Erfindung die Aufgabe zugrunde, ein Gleitmittel für thermoplastische Zusammensetzungen anzugeben, welches auf nachwachsenden Rohstoffen basiert und damit aus ökologischer Sicht vorteilhaft ist und zugleich eine besonders stark ausgeprägte interne Gleitmittelwirkung aufweist. Darüber hinaus sollte sich dieses Gleitmittel auf möglichst einfache Art und Weise ausgehend von nachwachsenden Rohstoffen herstellen lassen.

Auch sollte sich das Gleitmittel durch eine besonders gute Verträglichkeit insbesondere mit Polyvinylchlorid auszeichnen.

Des Weiteren sollte sich das Gleitmittel bzw. eine das Gleitmittel beinhaltende thermoplastische Zusammensetzung durch eine besonders hohe thermische Stabilität auszeichnen.

Einen Beitrag zur Lösung der eingangs genannten Aufgaben leistet eine thermoplastische Zusammensetzung beinhaltend
a) mindestens ein thermoplastisches Polymer,
b) mindestens ein Gleitmittel, sowie
c) gegebenenfalls weitere Zusatzstoffe,
wobei das mindestens eine Gleitmittel eine Reaktionsmischung ist, die durch die Umsetzung einer gesättigte Triglyceride beinhaltenden Komponente mit einem primären Alkohol erhältlich ist, wobei die Reaktionsmischung, welche neben dem Umesterungsprodukt (Ester aus Fettsäure und primärem Alkohol), auch Partialester von Glycerin, nicht umgesetzten primären Alkohol und nicht umgesetztes Triglycerid enthält; und wobei der primäre Alkohol ein Polyol mit mehr als zwei OH-Gruppen ist.

Überraschenderweise wurde gefunden, das gesättigte Triglyceride, mit primären Alkoholen umgeestert, als internes Gleitmittel bei der Verarbeitung von thermoplastischen Kunststoffen eingesetzt werden können, ohne die rheologischen Eigenschaften negativ zu beeinträchtigen. Zusätzlich verbessern diese Produkte die erreichbare Thermostabilität der Fertigprodukte. Die Gleitmittel bestehen aus einen hohen Anteil nachwachsender Rohstoffe und sind daher ökologisch vorteilhaft. Auch fallen bei der Herstellung dieser Gleitmittel keine Nebenprodukte an, da das Reaktionsprodukt der Umesterung direkt als Gleitmittel eingesetzt werden kann.

Die erfindungsgemäße Zusammensetzung beinhaltet als Komponente a) mindestens ein thermoplastisches Polymer. Ein geeignetes thermoplastisches Polymer ist beispielsweise ausgewählt aus der Gruppe bestehend aus Polyvinylchlorid (PVC), Polypropylen (PP), Polyethylen (PE), Polyethylenterephthalat (PET), Polylactat (PLA), Polycarbonat, Polystyrolen, Polyurethane, Poylether, Gummi, vorzugsweise Polyisopren, Kautschuk, insbesondere NBR-Kautschuk, Polybutadien, Copolymeren aus mindestens zwei der Vorstehenden, insbesondere Polyethylen/Polypropylen-Copolymere und Gemische aus mindestens zwei davon. Bevorzugt sind PE, PP, PVC, PET und PLA und am meisten bevorzugt ist PVC.

In einer erfindungsgemäßen Ausgestaltung schmilzt die erfindungsgemäße thermoplastische Zusammensetzung nicht unter 80°C, vorzugsweise bei oder unter 140°C und besonders bevorzugt bei oder unter 270°C. Meist liegen die erfindungsgemäß bevorzugten thermoplastischen Zusammensetzungen bei 350° oder weniger als Schmelze vor. In erfindungsgemäßen thermoplastischen Zusammensetzungen werden zu mindestens 50 Gew.-%, vorzugsweise zu mindestens 75 Gew.-% und besonders bevorzugt zu mindestens 90 Gew.-%, jeweils bezogen auf das Gesamtgewicht der thermoplastischen Zusammensetzung, thermoplastische Polymere mit ebensolchem Schmelzverhalten eingesetzt.

Eine erfindungsgemäße thermoplastische Zusammensetzung enthält vorzugsweise mindestens 50 Gew.-%, bezogen auf das Gesamtgewicht der thermoplastischen Zusammensetzung, mindestens eines thermoplastischen Polymeren. Die Obergrenze für den Gehalt an thermoplastischen Polymeren kann dabei beispielsweise bei 99,999 Gew.-%, jeweils bezogen auf das Gesamtgewicht der thermoplastischen Zusammensetzung, oder darunter liegen, beispielsweise bei 98 Gew.-%, 96 Gew.-%, 94 Gew.-%, 92 Gew.-%, 90 Gew.-% oder darunter, beispielsweise bei 85 Gew.-%, 80 Gew.-%, 75 Gew.-%, 70 Gew.-%, 65 Gew-%, 60 Gew.-% oder bei 55 Gew.-%. Es ist darüber hinaus ebenso möglich, dass eine erfindungsgemäße thermoplastische Zusammensetzung weniger an thermoplastischen Polymeren enthält, beispielsweise 45 Gew.-% oder weniger, beispielsweise 40 Gew.-% oder weniger, 35 Gew.-% oder weniger, 30 Gew.-% oder weniger, 25 Gew.-% oder weniger oder 20 Gew.-% oder weniger. In Abhängigkeit vom gewünschten Einsatzzweck kann die Untergrenze des Anteils an thermoplastischen Polymeren beispielsweise bei mehr als 15 Gew.-%, beispielsweise bei 16, 17, 18, 19 oder 20 Gew.-%, jeweils bezogen auf das Gesamtgewicht der thermoplastischen Zusammensetzung, liegen.

Eine erfindungsgemäße Zusammensetzung hat thermoplastische Eigenschaften. Dies schließt jedoch nicht aus, dass eine erfindungsgemäße thermoplastische Zusammensetzung einen Anteil an nicht thermoplastischen Polymerbestandteilen, beispielsweise einen Anteil an Duromeren (in der Literatur häufig auch Duroplasten genannt), enthält, der jedoch höchstens bei einer Menge liegt, bei der die thermoplastischen Eigenschaften der gesamten Zusammensetzung nicht derart nachteilig beeinflusst werden, dass eine thermoplastische Verarbeitung nicht oder nur Inkaufnahme gravierender Nachteile möglich ist.

Die erfindungsgemäße Zusammensetzung beinhaltet weiterhin als Komponente b) mindestens ein Gleitmittel, wobei das mindestens eine Gleitmittel eine Reaktionsmischung ist, die durch die Umsetzung einer gesättigte Triglyceride beinhaltenden Komponente mit einem primären Alkohol erhältlich ist, vorzugsweise erhalten worden ist. Diese Reaktionsmischung beinhaltet somit, neben Partialestern von Glycerin, einen Ester aus Fettsäuren und dem primären Alkohol.

Als Triglyceride beinhaltende Komponente werden vorzugsweise natürliche oder gehärte Öle und Fette pflanzlichen oder tierischen Ursprungs eingesetzt. Erfindungsgemäß bevorzugt ist der Einsatz von Ölen oder Fetten mit einem Anteil an Triglyceriden größer 50 Gew.-%, vorzugsweise größer 75 Gew.-% und besonders bevorzugt größer 90 Gew.-%, jeweils bezogen auf das Gesamtgewicht des Öls oder des Fettes.

Weiterhin ist es erfindungsgemäß bevorzugt, zur Herstellung des Gleitmittels eine Triglyceride beinhaltende Komponente einzusetzen, welche zu mindestens 75 Gew.-%, besonders bevorzugt zu mindestens 85 Gew.-% und am meisten bevorzugt zu mindestens 95 Gew.-%, jeweils bezogen auf das Gesamtgewicht der Triglycerin beinhaltenden Komponente, auf Ölen und Fetten mit einer vorzugsweise nach DIN 53241-1 bestimmten Iodzahl von weniger als 20, besonders bevorzugt weniger als 10 und darüber hinaus bevorzugt weniger als 5 basiert.

Derartige Fette und Öle können natürlich vorkommen oder durch Hydrierung von natürlichen Fetten und Ölen mit einem höheren Anteil an ungesättigten Fettsäuren und damit mit höherer Iodzahl erhalten werden. Ein Beispiel für natürliche vorkommende Fette und Öle mit Iodzahlen unter 20 kann das je nach Ernte und Anbaugebiet erhältliche Kokosöl sein, wobei naturgemäß die Fettsäurezusammensetzung dieses natürlichen Öls schwanken kann. Im Sinne der Erfindung ist nur solches natürliche Kokosöl geeignet, dass Iodzahlen unter 20, vorzugsweise unter 10 aufweist.

Weitere geeignete natürliche Fette und Öle mit Iodzahlen unter 20 sind erhältlich durch Hydrierung der Doppelbindungen der ungesättigten Fettsäuren in natürlichen Fetten und Ölen in an und sich bekannter Weise. Zur Hydrierung eignen sich als natürliche Öle und Fette Palmöl, Palmkernöl, Kokosöl, Olivenöl, Rüböl alter und neuer Züchtung, Sonnenblumenöl alter und neuer Züchtung, Leinöl, Erdnussöl, Baumwollsaatöl, Korianderöl, Meadowfoamöl, Lardöl, Rindertalg und Fischöl, Chaulmograaöl, Rizinusöl, insbesondere aber gehärteter Rindertalg. Sowohl die hydrierten Fette und Öle mit Jodzahlen unter 20 als auch die zugrunde liegenden natürlichen Fette und Öle, die noch hydriert werden müssen, sind im Handel erhältlich. Bevorzugt ist der Einsatz von Kokosöl, Palmkernöl, gehärteten Talg, gehärtetem Rüböl, gehärtetem Palmstearin, gehärtetem Rizinusöl oder einer Mischung aus mindestens zwei dieser Öle.

Unter einem primären Alkohol, welcher bei der Herstellung des Gleitmittels mit der Triglyceride beinhaltenden Komponente umgesetzt wird, wird vorzugsweise ein Alkohol verstanden, welcher mindestens eine Struktureinheit -CH₂-OH umfasst. Es handelt sich sich bei diesem primären Alkohol um einen Polyol mit mehr als zwei OH-Gruppen, beispielsweise um einen Polyol mit drei oder vier OH-Gruppen. Bevorzugte primäre Alkohole sind ausgewählt aus der Gruppe bestehend aus Trimethylolpropan, Pentaerythrit, Dipentaerythrit, und einer Mischung aus mindestens zwei davon.

Die bei der Umsetzung des primären Alkohols mit der Triglyceride beinhaltenden Komponente wird vorzugsweise eine Umesterung der Triglyceride angestrebt. Aus diesem Grund ist es bevorzugt, wenn die Umsetzung der gesättigte Triglyceride beinhaltenden Komponente mit dem primären Alkohol in Gegenwart einer Base als Katalysator erfolgt. Als Base eignen sich hierbei insbesondere Hydroxide, Oxide oder Carbonate eines Alkali- oder Erdalkalimetalls oder aber Alkali-Alkoxylate, beispielsweise Natriummethylat, Natriumethylat oder aber Alkali-Alkoxylate der vorstehend genannten primären Alkohole, wobei als Base besonders bevorzugt Verbindungen ausgewählt aus der Gruppe bestehend aus NaOH, KOH, LiOH oder einer Mischung aus mindestens zwei davon eingesetzt werden.

Weiterhin ist es im Zusammenhang mit der Herstellung des Gleitmittels bevorzugt, wenn die gesättigte Triglyceride beinhaltende Komponente mit dem primären Alkohol in einem Gewichtsverhältnis in einem Bereich von 50 : 50 bis 99 : 1, besonders bevorzugt in einem Bereich von 60 : 40 bis 98 : 2 und am meisten bevorzugt in einem Bereich von 70 : 30 bis 95 : 5 umgesetzt wird.

Weiterhin ist es im Zusammenhang mit der Herstellung des Gleitmittels bevorzugt, dass die Umsetzung der gesättigte Triglyceride beinhaltenden Komponente mit dem primären Alkohol bei einer Temperatur in einem Bereich von 100 bis 300°C, besonders bevorzugt in einem Bereich von 150 bis 280°C und am meisten bevorzugt in einem Bereich von 200 bis 250°C erfolgt.

Es hat sich nun als erfindungsgemäß besonders vorteilhaft erwiesen, wenn die nach der Umesterung erhaltene Reaktionsmischung, welche neben dem Umesterungsprodukt (Ester aus Fettsäure und primärem Alkohol) auch Partialester von Glycerin und nicht umgesetzten primären Alkohol, nicht umgesetztes Triglycerid sowie gegebenenfalls die als Katalysator eingesetzte Base enthält, ohne weitere Aufreinigung unmittelbar als Gleitmittelkomponente b) eingesetzt wird.

Das vorstehend beschriebene Gleitmittel ist in der erfindungsgemäßen thermoplastischen Zusammensetzung vorzugsweise in einer Menge in einem Bereich von 0,001 bis 10 Gew.-%, besonders bevorzugt von 0,1 bis 5 Gew.-% und am meisten bevorzugt von 0,3 bis 3 Gew.-%, jeweils bezogen auf das Gesamtgewicht der thermoplastischen Zusammensetzung, enthalten.

Weiterhin kann die erfindungsgemäße Zusammensetzung als Komponente c) weitere Zusatzstoffe enthalten, wobei der Zusatzstoff vorzugsweise ein Zusatzstoff ausgewählt aus der Gruppe bestehend aus Füllstoffen, Pigmenten, Stabilisatoren, weiteren Gleitmitteln, Weichmachern, Antiblockmitteln, Antibeschlagmitteln, Antistatika, Flammschutzmitteln, Treibmitteln, Fetten, Ölen, Antioxidantien, Säurefängern, Nukleierungsmitteln, Lösungsmitteln und eine Mischung aus mindestens zwei davon ist.

Als Pigmente eignen sich grundsätzlich alle anorganischen oder organischen Pigmente, die mit der thermoplastischen Zusammensetzung als solche und insbesondere mit der thermoplastischen Zusammensetzung im Rahmen der vorgesehenen Verarbeitung verträglich sind. Pigmente können der thermoplastischen Zusammensetzung eine Farbe verleihen, es sind jedoch ebenso Pigmente geeignet, die die thermoplastische Zusammensetzung weiß oder schwarz färben. Besonders geeignete Pigmente sind beispielsweise Titandioxid, Pigmente auf Zirconoxid Basis, Bariumsulfat, Zinkoxid (Zinkweiß) und Lithopone (Zinksulfit/Bariumsulfat), Ruß, Ruß-Titandioxid-Mischungen, Eisenoxidpigmente, Antimonoxid, Chromoxid, Spinelle wie Kobaltblau und Kobaltgrün, Cadmiumsulfit, Cadmiumselenit, Ultramarinblau oder organische Pigmente, wie Azopigmente, Phthalocyaninpigmente, Chinacridonpigmente, Perylenpigmente, Diketo-Pyrrolopyrrolpigmente oder Anthrachinonpigmente.

Als Füllstoffe eignen sich insbesondere Kalziumcarbonat, Dolomit, Gips, Wollastonit, Magnesiumoxid, Magnesiumhydroxid, Silikate, China-Clay, Talg, Glasfasern, Glaskugeln, Holzkugeln, Glimmer, Metalloxide oder Metallhydroxide, Ruß, Grafit, Gesteinsmehl, Schwerspat, Glasfasern, Talg, Kaolin oder Kreide oder Metallsulfate, beispielsweise Schwermetallsulfate die neben einer füllenden oder pigmentierenden Wirkung beispielsweise auch eine stabilisierende Wirkung auf die thermoplastische Zusammensetzung ausüben können.

Der Anteil an Pigmenten oder Füllstoffen oder Gemischen aus einem oder mehreren Pigmenten und einem oder mehreren Füllstoffen an der gesamten thermoplastischen Zusammensetzung kann bis zu etwa 50 Gew.-%, bezogen auf die thermoplastische Zusammensetzung, betragen. Es ist erfindungsgemäß vorgesehen, dass eine thermoplastische Zusammensetzung nur geringe Mengen an Pigmenten oder Füllstoffen, beispielsweise 0,5 bis etwa 10 oder 1 bis etwa 5 Gew.-% enthält. Es ist jedoch ebenso möglich, dass eine erfindungsgemäße thermoplastische Zusammensetzung größere Mengen an Pigmenten oder Füllstoffen oder deren Gemischen, beispielsweise etwa 10 bis etwa 50 Gew.-% oder etwa 20 bis etwa 40 Gew.-%, jeweils bezogen auf das Gesamtgewicht der thermoplastischen Zusammensetzung, enthält.

Stabilisatoren bewahren Kunststoffe wie PVC davor, sich bei hohen Temperaturen zu zersetzen oder chemisch zu verändern. Sie verbessern dadurch die Witterungsbeständigkeit. Eingesetzt werden beispielsweise Verbindungen auf der Basis von Blei, Calcium, Zink, Barium und Zinn.

Weitere Gleitmittel dienen beispielsweise dazu, die Verarbeitung von Polymeren durch Verminderung der Friktion zwischen den Polymerketten und die Herabsetzung der Wandhaftung der Schmelze noch weiter zu erleichtern. Häufig verwendete weitere Gleitmittel sind Metallseifen, wie Blei- und Calciumstearate und - laurate, die gleichzeitig als Co-Stabilisator wirken können.

Weichmacher verleihen dem Kunststoff Geschmeidigkeit und Flexibilität. Viele Weichmacher gehören zur Gruppe der Phthalate (DEHP, DINP und DIDP), sowie der Adipate und Citrate.

Antibeschlagmittel dienen dazu, die Bildung von Trübungen, hervorgerufen beispielsweise durch Wassertröpfchen, an der Oberfläche zu verhindern. Derartige Antibeschlagmittel sind beispielsweise in DE 10 2004 038 980 A1 und Plastics Additives Handbook, 5th Edition, Hanser Verlag, S. 609 bis 626 offenbart und durch die Emery Oleochemicals GmbH zu beziehen.

Antiblockmittel sind Additive, die das Verkleben (*"Blocken"*) beschichteter Flächen miteinander oder mit Substraten (z.B. beim Stapeln oder Verpacken) verhindern oder reduzieren. Je nach Ablüftzeit, Trocknungsgrad, Schichtdicke, Druck oder Temperatur bei einer bestimmten Belastung muss die Auswahl geeigneter Trennmittel erfolgen, die in der Regel dem Beschichtungsstoff zugesetzt werden und während der Trocknungsphase an die Oberfläche gelangen. Hierfür finden beispielsweise Paraffin, Polyethylenwachs, Wachsester, Siliconöle, Stearate, modifizierte Kieselsäuren und Talkum Verwendung.

Als Lösungsmittel können Wasser oder organische Lösungsmittel wie Alkohole, wie Polyglycol, insbesondere Polyethylenglycol oder Polypropylenglcol oder eine Mischung der vorstehend genannten, eingesetzt werden.

Gemäß einer besonderen Ausführungsform der erfindungsgemäßen thermoplastischen Zusammensetzung enthält diese
a) 50 bis 99,999 Gew.-%, besonders bevorzugt 60 bis 94,9 Gew.-% und am meisten bevorzugt 70 bis 89,7 Gew.-% des mindestens einen thermoplastischen Polymers,
b) 0,001 bis 10 Gew.-%. besonders bevorzugt 0,1 bis 5 Gew.-% und am meisten bevorzugt 0,3 bis 3 Gew.-% des mindestens einen Gleitmittels und
c) 0 bis 49,999 Gew.-%, besonders bevorzugt 5 bis 39,9 Gew.-% und am meisten bevorzugt 10 bis 29,7 Gew.-% der weiteren Zusatzstoffe,
jeweils bezogen auf das Gesamtgewicht der thermoplastischen Zusammensetzung, enthält, wobei die Summe der Komponenten a) bis c) 100 Gew.-% beträgt. Einen Beitrag zur Lösung der eingangs genannten Aufgaben leistet auch ein Verfahren zur Herstellung einer thermoplastischen Zusammensetzung durch in Kontakt bringen von mindestens der folgenden Zusammensetzungskomponenten
A) 50 bis 99,999 Gew.-%, besonders bevorzugt 60 bis 94,9 Gew.-% und am meisten bevorzugt 70 bis 89,7 Gew.-%, jeweils bezogen auf das Gesamtgewicht der thermoplastischen Zusammensetzung, mindestens eines thermoplastischen Polymers;
B) 0,001 bis 10 Gew.-%, besonders bevorzugt 0,1 bis 5 Gew.-% und am meisten bevorzugt 0,3 bis 3 Gew.-%, jeweils bezogen auf das Gesamtgewicht der thermoplastischen Zusammensetzung, mindestens des einen Gleitmittels und
C) 0 bis 49,999 Gew.-%, besonders bevorzugt 5 bis 39,9 Gew.-% und am meisten bevorzugt 10 bis 29,7 Gew.-%, jeweils bezogen auf das Gesamtgewicht der thermoplastischen Zusammensetzung, mindestens eines Zusatzstoffes, wobei die Summe der Komponenten A) bis C) 100 Gew.-% beträgt und wobei als thermoplastische Polymere, als Gleitmittel und als Zusatzstoffe diejenigen Verbindungen bevorzugt sind, die bereits eingangs im Zusammenhang mit der erfindungsgemäßen thermoplastischen Zusammensetzung als bevorzugte thermoplastische Polymer, Gleitmittel und Zusatzstoffe genannt wurden.

Das in Kontakt bringen der Komponenten A), B) und C) kann im einfachsten Fall durch das einfache Trockenvermischen der drei Komponenten erfolgen (Trockenmischverfahren), wobei das mindestens eine thermoplastische Polymer beispielsweise als Granulat eingesetzt werden kann. Denkbar ist aber auch, das mindestens eine thermoplastische Polymer über seinen Schmelzpunkt hinaus zu erhitzen und somit zu schmelzen und sodann die Komponenten B) und C) in die Schmelze einzuarbeiten (Schmelzmischverfahren). Im Anschluss daran kann die Mischung abgekühlt und gegebenenfalls in eine granuläre Form überführt werden.

Einen Beitrag zur Lösung der eingangs genannten Aufgaben leistet auch eine durch das vorstehend beschriebene Verfahren erhältliche thermoplastische Zusammensetzung, wobei es bevorzugt ist, dass diese thermoplastische Zusammensetzung die gleichen Eigenschaften aufweist wie die eingangs beschriebene, erfindungsgemäße thermoplastische Zusammensetzung.

Einen Beitrag zur Lösung der eingangs genannten Aufgaben leistet weiterhin ein Verfahren zur Herstellung eines Formkörpers, beinhaltend die Verfahrensschritte:
I) das Bereitstellen einer erfindungsgemäßen thermoplastischen Zusammensetzung oder einer durch das erfindungsgemäße Verfahren erhältlichen thermoplastischen Zusammensetzung;
II) das Erhitzen der thermoplastischen Zusammensetzung auf die Glasübergangstemperatur des thermoplastischen Polymers oder auf eine Temperatur oberhalb der Glasübergangstemperatur des thermoplastischen Polymers;
III) die Herstellung eines Formkörpers aus der im Verfahrensschritt II) hergestellten, erhitzten, thermoplastischen Zusammensetzung.

Im Schritt I) des erfindungsgemäßen Verfahrens zur Herstellung eines Formkörpers wird zunächst eine erfindungsgemäße thermoplastische Zusammensetzung oder einer durch das erfindungsgemäße Verfahren erhältlich thermoplastische Zusammensetzung bereitgestellt.

Sodann wird im Verfahrensschritt II) die thermoplastische Zusammensetzung auf die Glasübergangstemperatur des thermoplastischen Polymers oder auf einer Temperatur oberhalb der Glasübergangstemperatur des thermoplastischen Polymers erhitzt. In diesem Zusammenhang ist es wiederum bevorzugt, dass das Erhitzen der thermoplastischen Zusammensetzung auf eine Temperatur in einem Bereich von 5 Grad unterhalb der Glasübergangstemperatur (Tg) bis 100°C oberhalb der Glasübergangstemperatur des eingesetzten, thermoplastischen Polymers, besonders bevorzugt auf eine Temperatur in einem Bereich von 1 Grad unterhalb der Glasübergangstemperatur (Tg) bis 50°C oberhalb der Glasübergangstemperatur des eingesetzten, thermoplastischen Polymers und am meisten bevorzugt auf eine Temperatur in einem Bereich von 1 Grad oberhalb der Glasübergangstemperatur (Tg) bis 20°C oberhalb der Glasübergangstemperatur des eingesetzten, thermoplastischen Polymers erfolgt, wobei jedoch auch hier die obere Grenze des Temperaturbereiches im wesentlichen von der Zersetzungstemperatur des eingesetzten, thermoplastischen Polymers begrenzt wird.

Grundsätzlich können die Verfahrensschritte I) und II) zeitgleich oder hintereinander durchgeführt werden. Eine gleichzeitige Durchführung der Verfahrensschritte I) und II) ist beispielsweise dann sinnvoll, wenn die thermoplastische Zusammensetzung mittels eines Schmelzmischverfahrens hergestellt wird. Hier kann es gegebenenfalls vorteilhaft sein, die durch das Schmelzmischverfahren hergestellte Zusammensetzung unmittelbar in einen Formkörper zu überführen. Eine nacheinander erfolgende Durchführung der Verfahrensschritte I) und II) ist beispielsweise dann sinnvoll, wenn die thermoplastische Zusammensetzung mittels eines Trockenmischverfahrens hergestellt wird oder aber wenn die thermoplastische Zusammensetzung zwar mittels eines Schmelzmischverfahrens hergestellt wird, jedoch nicht unmittelbar nach der Herstellung der Bildung eines Formkörpers unterzogen wird sondern vielmehr zunächst abgekühlt wird.

Im Verfahrensschritt III) des erfindungsgemäßen Verfahrens zur Herstellung eines Formkörpers wird aus der im Verfahrensschritt II) hergestellten, erhitzten, thermoplastischen Zusammensetzung ein Formkörper hergestellt, bei dem es sich vorzugsweise um ein Behältnis, eine Folie, eine Faser, ein Profil oder ein Rohr handelt. Als Verfahren zur Herstellung eines Formkörpers kommen insbesondere das Spritzgießen, das Extrusionsformen, das Kompressionsformen, das Schichtformen, das Laminierungsformen, das Hohlformen, das Vakuumformen und das Transferformen in Betracht, wobei das Spritzgießen besonders bevorzugt ist.

Weiterhin entspricht es einer Ausgestaltung des erfindungsgemäßen Verfahrens zur Herstellung eines thermoplastischen Formkörpers, dass in mindestens einem weiteren Verfahrensschritt IV) mindestens ein Teilbereich des in Verfahrensschritt III) erhaltenen Formkörpers als Formkörperrohling dient und in seinem Massequerschnitt gegenüber verringert wird. Bei dem Massequerschnitt handelt es sich um den Querschnitt eines Bereiches des Formkörpers, der massiv aus der erfindungsgemäßen thermoplastischen Formmasse besteht. Beispielsweise bei Behältern oder Gebinden stellt der Massequerschnitt die Dicke eine Wandung dieser Behälter oder Gebinde dar. Bei eher faden- oder strangförmig ausgebildeten Formkörpern stellt der Massequerschnitt die Dicke dieser Fäden oder Stränge dar.

Bei eher flächigen Gebilden wie Platten, Lagen, Bahnen, Filmen oder Folien stellt der Massequerschnitt die Stärke dieser flächigen Gebilde dar. Für das Verringern des Massequerschnitts kommen grundsätzlich alle dem Fachmann hierzu bekannten und geeigneten Methoden in Betracht. Hierunter fallen beispielsweise das Strecken in eine oder zwei Richtungen, Ziehen in eine oder zwei Richtungen, Schleudern oder Blasen, die jeweils vorzugsweise bei erhöhten Temperaturen erfolgen, bei denen die erfindungsgemäße thermoplastische Zusammensetzung so weich oder gar flüssig ist, dass ein Strecken, Ziehen Schleudern oder Blasen erfolgen kann. Der Teilbereich, in dem die Querschnittsverringerung erfolgt, macht vorzugsweise mindestens 50% und besonders bevorzugt mindesten 80% des in Schritt III) erhaltenen Formkörpers aus. Allgemein erfolgt das Strecken oder Ziehen, wenn aus dem in Schritt III) erhaltenen Formkörper eine Faser erhalten werden soll. Bei der Herstellung von Folien kann zum einen das Ziehen oder Strecken in eine oder mehrere Dimensionen erfolgen. So kann die aus einem Extruder laufende Bahn mit einer im Vergleich zu der Austrittsgeschwindigkeit aus dem Extruder höheren Geschwindigkeit auf eine Rolle gezogen werden. Soll hingegen ein Behälter oder Gebinde erhalten werden, so wird außer dem Strecken, Ziehen und Schleudern vornehmlich das Blasen in Schritt IV) eingesetzt. Hierbei erfolgt die Massequerschnittsverringerung durch das anlegen eines Gasdrucks. Der Gasdruck wird allgemein so gewählt, dass die meist mindestens auf Glasübergangstemperatur erhitzte thermoplastische Zusammensetzung des in Schritt In) erhaltenen Formkörpers gedehnt werden kann. In der Regel wird die Dehnung durch die Verwendung eines die Endform des Formkörpers habende Form begrenzt. Es weiterhin möglich, dass zwei oder mehrere der Verfahrenschritte I) bis IV) durch weitere Verfahrensschritte ergänzt werden und/oder zumindest zeitlich überlappende verlaufen. Dieses gilt insbesondere für die Verfahrensschritte III) und IV).

Einen Beitrag zur Lösung der eingangs genannten Aufgaben leistet auch ein durch das vorstehend beschriebene Verfahren erhältlicher Formkörper.

Einen Beitrag zur Lösung der eingangs genannten Aufgaben leistet schließlich auch die Verwendung der im Zusammenhang mit der erfindungsgemäßen thermoplastischen Zusammensetzung beschriebenen Reaktionsmischung als Gleitmittel in thermoplastischen Zusammensetzungen. Als thermoplastische Polymere und als Gleitmittel sind wiederum diejenigen Verbindungen bevorzugt, die bereits eingangs im Zusammenhang mit der erfindungsgemäßen thermoplastischen Zusammensetzung als bevorzugte thermoplastische Polymer und Gleitmittel genannt wurden.

Die Lösung wird nun anhand nicht limitierender Beispiele näher erläutert.

### Beispiel 1: Umesterung von gehärtetem Talg mit Trimethylolpropan

In einem Glaskolben wurden 518,3 g gehärteter Talg (Iodzahl < 1), 81,7 g Trimethylolpropan und 0.061 g LiOH × H₂O vorgelegt und unter Rühren auf 240°C erhitzt. Nach 2,5 h Reaktionszeit wurde abgekühlt.

Farbe 1" Lov. Gelb = 0,5, Lov. Rot = 0.2 (Lov. = Farbzahl nach Lovibond) Säurezahl = 0.27, Verseifungszahl = 171,2,

### Beispiel 2: Umesterung von gehärtetem Talg mit Pentaerythritol

In einem Glaskolben wurden 517,2 g gehärteter Talg (Iodzahl < 1), 82,8 g Pentaerythritol und 0.06 g LiOH × H₂O vorgelegt und unter Rühren auf 240°C erhitzt. Nach 2 h Reaktionszeit wurde abgekühlt.

Farbe 1" Lov. Gelb = 0,7, Lov. Rot = 0.3

Säurezahl = 0.22, Verseifungszahl = 170,0

### Beispiel 3: Umesterung von geh. Talg mit Triethylenglykol

In einem Glaskolben wurden 510,0 g gehärteter Talg (Iodzahl < 1), 90,0 g Triethylenglykol und 0.06 g LiOH × H₂O vorgelegt und unter Rühren auf 240°C erhitzt. Nach 2,5 h Reaktionszeit wurde abgekühlt.
Farbe 1" Lov. Gelb = 3,8, Lov. Rot = 1,4
Säurezahl = 0.8, Verseifungszahl = 171,3

### Beispiele 4-7: Herstellung einer erfindungsgemäßen thermoplastischen Zusammensetzung

Aus PVC-Pulver und verschiedenen Additiven wurde in einem Mischer der Firma Henschel ein Dry-Blend hergestellt (Materialmenge = 3 kg, Heiztemperatur = 120°C, anschließendes Abkühlen), die Zusammensetzungen sind der nachfolgenden Tabelle zu entnehmen.

| Beispiel | 4 | 5 | 6 | 7 |
|---|---|---|---|---|
| PVC Evipol SH 6520 | 100 | 100 | 100 | 100 |
| Bleisulfat 3-basisch | 3 | 3 | 3 | 3 |
| Bleistearat 28% | 0,5 | 0,5 | 0,5 | 0,5 |
| Calciumstearat | 0,5 | 0,5 | 0,5 | 0,5 |
| Distearylphthalat | 1 | | | |
| Umesterungsprodukt aus Beispiel 1 | | 1 | | |
| Umesterungsprodukt aus Beispiel 2 | | | 1 | |
| Umesterungsprodukt aus Beispiel 3 | | | | 1 |

Die Beispiele 5 bis 7 sind erfindungsgemäß

### Herstellung von Flachbändern als Formkörper

Die Dry-Blends aus den Beispielen 4 bis 7 wurden auf einem Doppelschneckenextruder der Firma. Weber zu einem Flachband extrudiert. (Parameter der Extrusion: Drehzahl = 15 UpM (B1-B3), 25 UpM; Temperatur = 180°C). Als Maß für die Gleitmittelwirksamkeit wurde die Leistungsaufnahme des Extruders, die Maschinenbelastung (in %) und der Massedruck (in bar) ausgewertet.

| Beispiel | Maschinenbelastung (%) | Massedruck (bar) |
|---|---|---|
| 4 | 49 | 382 |
| 5 | 46 | 362 |
| 6 | 48 | 365 |
| 7 | 49 | 376 |

Die erfindungsgemäßen Produkte sind vergleichbar mit dem Standardgleitmittel Distearylphthalat und senken den Massedruck im Extruder ohne die Maschinenbelastung zu verändern.

Von den Extrudaten wurde die thermische Stabilität nach EN 60811-3-2: 1995 Abs. 9 (Kongorot-Test) bei 200°C bestimmt.

| Beispiel | Stabilität [min] |
|---|---|
| 4 | 46 |
| 5 | 51 |
| 6 | 51 |
| 7 | 51 |

Die erfindungsgemäßen eingesetzten Gleitmittel führen zu einer besseren Thermostabilität als der Standard.

## Patentansprüche

1. Eine thermoplastische Zusammensetzung beinhaltend
a) mindestens ein thermoplastisches Polymer,
b) mindestens ein Gleitmittel, sowie
c) gegebenenfalls weitere Zusatzstoffe,
wobei das mindestens eine Gleitmittel eine Reaktionsmischung ist, die durch die Umsetzung einer gesättigte Triglyceride beinhaltenden Komponente mit einem primären Alkohol erhältlich ist,
wobei die Reaktionsmischung, welche neben dem Umesterungsprodukt (Ester aus Fettsäure und primärem Alkohol), auch Partialester von Glycerin, nicht umgesetzten primären Alkohol und nicht umgesetztes Triglycerid enthält; und wobei der primäre Alkohol ein Polyol mit mehr als zwei OH-Gruppen ist.

2. Die thermoplastische Zusammensetzung nach Anspruch 1, wobei die Triglycerid beinhaltende Komponente ausgewählt ist aus der Gruppe bestehend aus Kokosöl, Palmkernöl, gehärteter Talg, gehärtetes Ruböl, gehärtetes Palmstearin, gehärtetes Rizinusöl und einer Mischung aus mindestens zwei davon.

3. Die thermoplastische Zusammensetzung nach Anspruch 1 oder 2, wobei die Triglyceride beinhaltende Komponente zu mindestens 75 Gew.-%, bezogen auf das Gesamtgewicht der Triglyceride beinhaltenden Komponente, auf Ölen und Fetten mit einer Jodzahl von weniger als 20 basiert.

4. Die thermoplastische Zusammensetzung nach einem der vorhergehenden Ansprüche, wobei der primäre Alkohol ausgewählt ist aus der Gruppe bestehend aus Trimethylolpropan, Pentaerythrit, Dipentaerythrit und einer Mischung aus mindestens zwei davon.

5. Die thermoplastische Zusammensetzung nach einem der vorhergehenden Ansprüche, wobei die Umsetzung der gesättigte Triglyceride beinhaltenden Komponente mit dem primären Alkohol in Gegenwart einer Base erfolgt.

6. Die thermoplastische Zusammensetzung nach Anspruch 5, wobei die Base ein Hydroxid, ein Oxid oder ein Carbonat eines Alkali- oder Erdalkalimetalls oder ein Alkali-Alkoxylat ist.

7. Die thermoplastische Zusammensetzung nach Anspruch 6, wobei die Base ausgewählt ist aus der Gruppe bestehend aus NaOH, KOH, LiOH und einer Mischung aus mindestens zwei davon.

8. Die thermoplastische Zusammensetzung nach einem der vorhergehenden Ansprüche, wobei die gesättigte Triglyceride beinhaltende Komponente mit dem primären Alkohol in einem Gewichtsverhältnis in einem Bereich von 50 : 50 bis 99 : 1 umgesetzt wird.

9. Die thermoplastische Zusammensetzung nach einem der vorhergehenden Ansprüche, wobei die Umsetzung der gesättigte Triglyceride beinhaltenden Komponente mit dem primären Alkohol bei einer Temperatur in einem Bereich von 100 bis 300°C erfolgt.

10. Die thermoplastische Zusammensetzung nach einem der vorhergehenden Ansprüche, wobei das thermoplastische Polymer ausgewählt ist aus der Gruppe bestehend aus Polyvinylchlorid, Polypropylen, Polyethylen, Polyethylenterephthalat, Polylactat, Polycarbonat, Polystyrol, Polyurethan, Polyether, Gummi und Copolymere aus mindestens zwei der Vorstehenden.

11. Die thermoplastische Zusammensetzung nach einem der vorhergehenden Ansprüche, wobei als Zusatzstoff mindestens ein Zusatzstoff ausgewählt aus der Gruppe bestehend aus Füllstoffen, Pigmenten, Stabilisatoren, weiteren Gleitmitteln, Weichmachern, Antiblockmitteln, Antibeschlagmitteln, Antistatika, Flammschutzmitteln, Treibmitteln, Fetten, Ölen, Antioxidantien, Säurefängern, Nukleierungsmitteln, Lösungsmitteln und eine Mischung aus mindestens zwei davon enthalten ist.

12. Die thermoplastische Zusammensetzung nach einem der vorhergehenden Ansprüche, wobei die Zusammensetzung das Gleitmittel in einer Menge von etwa 0,001 bis etwa 10 Gew.-%, bezogen auf das Gesamtgewicht der thermoplastischen Zusammensetzung, enthält.

13. Die thermoplastische Zusammensetzung nach einem der vorhergehenden Ansprüche, wobei die thermoplastische Zusammensetzung
a) mindestens 50 bis 99,999 Gew.-% des mindestens thermoplastischen Polymers,
b) 0,001 bis 10 Gew.-% des mindestens einen Gleitmittels und
c) 0 bis 49,999 Gew.-% der weiteren Zusatzstoffe,
jeweils bezogen auf das Gesamtgewicht der thermoplastischen Zusammensetzung, enthält, wobei die Summe der Komponenten a) bis c) 100 Gew.-% beträgt.

14. Ein Verfahren zur Herstellung einer thermoplastischen Zusammensetzung durch in Kontakt bringen von mindestens der folgenden Zusammensetzungskomponenten
A) mindestens 50 bis 99,999 Gew.-%, bezogen auf das Gesamtgewicht der thermoplastischen Zusammensetzung, mindestens eines thermoplastischen Polymers;
B) 0,001 bis 10 Gew.-%, bezogen auf das Gesamtgewicht der thermoplastischen Zusammensetzung, mindestens eines Gleitmittels wie in den Ansprüchen 1-13 definiert ; und
C) 0 bis 49,999 Gew.-%, bezogen auf das Gesamtgewicht der thermoplastischen Zusammensetzung, mindestens eines Zusatzstoffes
wobei die Summe der Komponenten A) bis C) 100 Gew.-% beträgt.

15. Eine thermoplastische Zusammensetzung, erhältlich durch das Verfahren nach Anspruch 14.

16. Ein Verfahren zur Herstellung eines Formkörpers, beinhaltend die Verfahrensschritte:
I) das Bereitstellen einer thermoplastischen Zusammensetzung nach einem der Ansprüche 1 bis 13 oder 15,
II) das Erhitzen der thermoplastischen Zusammensetzung auf die Glasübergangstemperatur des thermoplastischen Polymers oder auf eine Temperatur oberhalb der Glasübergangstemperatur des thermoplastischen Polymers;
III) die Herstellung eines Formkörpers aus der im Verfahrensschritt II) hergestellten, erhitzten, thermoplastischen Zusammensetzung.

17. Das Verfahren nach Anspruch 16, wobei in einem weiteren Verfahrensschritt IV) mindestens ein Teilbereich des in Verfahrens schritt III) erhaltenen Formkörpers in seinem Massequerschnitt gegenüber Verfahrensschritt III) verringert wird.

18. Das Verfahren nach einem der Ansprüche 16 oder 17, wobei der Formkörper ausgewählt ist aus einer Gruppe bestehend aus einem Behältnis, einer Folie, einer Faser, einem Profil und einem Rohr.

19. Ein Formkörper, erhältlich durch ein Verfahren nach einem der Ansprüche 16 bis 18.

20. Die Verwendung einer Reaktionsmischung, die durch die Umsetzung einer gesättigte Triglyceride beinhaltenden Komponente mit einem primären Alkohol erhältlich ist, als Gleitmittel in thermoplastischen Zusammensetzungen;
wobei die Reaktionsmischung, welche neben dem Umesterungsprodukt (Ester aus Fettsäure und primärem Alkohol), auch Partialester von Glycerin, nicht umgesetzten primären Alkohol und nicht umgesetztes Triglycerid enthält; und wobei der primäre Alkohol ein Polyol mit mehr als zwei OH-Gruppen ist.

## Claims

1. A thermoplastic composition including
a) at least one thermoplastic polymer,
b) at least one lubricant, and also
c) optionally other additional substances,
wherein the at least one lubricant is a reaction mixture which is obtainable via the reaction of a primary alcohol with a component including saturated triglycerides,
wherein the reaction mixture, which in addition to the transesterification product (ester of fatty acid and primary alcohol), also comprises partial ester(s) of glycerol, unreacted primary alcohol and unreacted triglyceride, and
wherein the primary alcohol is a polyol having more than two OH groups.

2. The thermoplastic composition according to Claim 1, wherein the component including triglyceride is selected from the group consisting of coconut oil, palm kernel oil, hardened tallow, hardened rapeseed oil, hardened palm stearin, hardened castor oil and a mixture of at least two thereof.

3. The thermoplastic composition according to Claim 1 or 2, wherein the component including triglycerides is based to an extent of at least 75% by weight, based on the total weight of the component including triglycerides, on oils and fats with an iodine number of less than 20.

4. The thermoplastic composition according to any of the preceding claims, wherein the primary alcohol is selected from the group consisting of trimethylol-propane, pentaerythritol, dipentaerythritol and a mixture of at least two thereof.

5. The thermoplastic composition according to any of the preceding claims, wherein the reaction of the primary alcohol with the component including saturated triglycerides takes place in the presence of a base.

6. The thermoplastic composition according to Claim 5, wherein the base is a hydroxide, an oxide or a carbonate of an alkali metal or of an alkaline earth metal or is an alkali metal alkoxylate.

7. The thermoplastic composition according to Claim 6, wherein the base is selected from the group consisting of NaOH, KOH, LiOH and a mixture of at least two thereof.

8. The thermoplastic composition according to any of the preceding claims, wherein the component including saturated triglycerides is reacted with the primary alcohol in a ratio by weight in the range from 50 : 50 to 99 : 1.

9. The thermoplastic composition according to any of the preceding claims, wherein the reaction of the primary alcohol with the component including saturated triglycerides takes place at a temperature in the range from 100 to 300°C.

10. The thermoplastic composition according to any of the preceding claims, wherein the thermoplastic polymer is selected from the group consisting of polyvinyl chloride, polypropylene, polyethylene, polyethylene terephthalate, polylactate, polycarbonate, polystyrene, polyurethane, polyether, rubber and copolymers of at least two of the above.

11. The thermoplastic composition according to any of the preceding claims, wherein additional substance present comprises at least one additional substance selected from the group consisting of fillers, pigments, stabilizers, further lubricants, plasticizers, antiblocking agents, antifogging agents, antistatic agents, flame retardants, blowing agents, fats, oils, antioxidants, acid scavengers, nucleating agents, solvents and a mixture of at least two thereof.

12. The thermoplastic composition according to any of the preceding claims, wherein the composition comprises an amount of about 0.001 to about 10% by weight of the lubricant, based on the total weight of the thermoplastic composition.

13. The thermoplastic composition according to any of the preceding claims, wherein the thermoplastic composition comprises
a) at least 50 to 99.999% by weight of the at least one thermoplastic polymer,
b) 0.001 to 10% by weight of the at least one lubricant and
c) 0 to 49.999% by weight of the further additional substances,
based in each case on the total weight of the thermoplastic composition, wherein the entirety of components a) to c) is 100% by weight.

14. A process for the production of a thermoplastic composition by bringing at least the following composition components
A) at least 50 to 99.999% by weight, based on the total weight of the thermoplastic composition, of at least one thermoplastic polymer;
B) 0.001 to 10% by weight, based on the total weight of the thermoplastic composition, of at least one lubricant as defined in Claims 1-13; and
C) 0 to 49.999% by weight, based on the total weight of the thermoplastic composition, of at least one additional substance
into contact, wherein the entirety of components A) to C) is 100% by weight.

15. A thermoplastic composition obtainable via the process according to Claim 14.

16. A process for the production of a moulding, including the steps of:
I) the provision of a thermoplastic composition according to any of Claims 1 to 13 or 15,
II) the heating of the thermoplastic composition to the glass transition temperature of the thermoplastic polymer or to a temperature above the glass transition temperature of the thermoplastic polymer;
III) the production of a moulding from the heated thermoplastic composition produced in step II).

17. The process according to Claim 16, wherein, in a further step IV), the mass cross section of at least one sub-region of the moulding obtained in step III) is reduced in comparison with step III).

18. The process according to Claim 16 or 17, wherein the moulding is selected from a group consisting of a container, a foil, a fibre, a profile and a tube.

19. A moulding obtainable via a process according to any of Claims 16 to 18.

20. The use of a reaction mixture obtainable via the reaction of a primary alcohol with a component including saturated triglycerides, as lubricant in thermoplastic compositions;
wherein the reaction mixture, which in addition to the transesterification product (ester of fatty acid and primary alcohol), also comprises partial ester(s) of glycerol, unreacted primary alcohol and unreacted triglyceride, and
wherein the primary alcohol is a polyol having more than two OH groups.

## Revendications

1. Composition thermoplastique comportant
a) au moins un polymère thermoplastique,
b) au moins un lubrifiant, ainsi que
c) éventuellement d'autres additifs,
ledit au moins un lubrifiant étant un mélange réactionnel qui peut être obtenu par mise en réaction d'un composant, comportant des triglycérides saturés, avec un alcool primaire,
le mélange réactionnel, qui outre le produit de transestérification (ester d'acide gras et d'alcool primaire), contenant également des esters partiels de glycérol, de l'alcool primaire n'ayant pas réagi et du triglycéride n'ayant pas réagi ; et
l'alcool primaire étant un polyol comportant plus de deux groupes OH.

2. Composition thermoplastique selon la revendication 1, dans laquelle le composant comportant des triglycérides est choisi dans le groupe constitué par l'huile de coprah, l'huile de palmiste, le suif hydrogéné, l'huile de navette hydrogénée, la stéarine de palme hydrogénée, l'huile de ricin hydrogénée et un mélange d'au moins deux de ceux-ci.

3. Composition thermoplastique selon la revendication 1 ou 2, dans laquelle le composant comportant des triglycérides est à raison d'au moins 75 % en poids, par rapport au poids total du composant comportant des triglycérides, à base d'huiles et de graisses ayant un indice d'iode de moins de 20.

4. Composition thermoplastique selon l'une quelconque des revendications précédentes, dans laquelle l'alcool primaire est choisi dans le groupe constitué par le triméthylolpropane, le pentaérythritol, le dipentaérythritol et un mélange d'au moins deux de ceux-ci.

5. Composition thermoplastique selon l'une quelconque des revendications précédentes, dans laquelle la réaction du composant comportant des triglycérides saturés avec l'alcool primaire s'effectue en présence d'une base.

6. Composition thermoplastique selon la revendication 5, dans laquelle la base est un hydroxyde, un oxyde ou un carbonate d'un métal alcalin ou alcalino-terreux ou un alcoxylate de métal alcalin.

7. Composition thermoplastique selon la revendication 6, dans laquelle la base est choisie dans le groupe constitué par NaOH, KOH, LiOH et un mélange d'au moins deux de ceux-ci.

8. Composition thermoplastique selon l'une quelconque des revendications précédentes, dans laquelle on fait réagir le composant comportant des triglycérides saturés avec l'alcool primaire en un rapport pondéral dans une plage de 50 : 50 à 99 : 1.

9. Composition thermoplastique selon l'une quelconque des revendications précédentes, dans laquelle la réaction du composant comportant des triglycérides saturés avec l'alcool primaire s'effectue à une température dans une plage de 100 à 300 °C.

10. Composition thermoplastique selon l'une quelconque des revendications précédentes, dans laquelle le polymère thermoplastique est choisi dans le groupe constitué par le poly(chlorure de vinyle), le polypropylène, le polyéthylène, le poly(éthylènetéréphtalate), le polylactate, le polycarbonate, le polystyrène, le polyuréthane, des polyéthers, le caoutchouc et des copolymères d'au moins deux des polymères précédents.

11. Composition thermoplastique selon l'une quelconque des revendications précédentes, dans laquelle comme additif est contenu au moins un additif choisi dans le groupe constitué par des charges, des pigments, des stabilisants, d'autres lubrifiants, des plastifiants, des agents anti-adhérence de contact, des agents anti-exsudation, des agents antistatiques, des agents ignifuges, des agents d'expansion, des graisses, des huiles, des antioxydants, des capteurs d'acide, des agents de nucléation, des solvants et un mélange d'au moins deux de ceux-ci.

12. Composition thermoplastique selon l'une quelconque des revendications précédentes, dans laquelle la composition contient le lubrifiant en une quantité d'environ 0,001 à environ 10 % en poids, par rapport au poids total de la composition thermoplastique.

13. Composition thermoplastique selon l'une quelconque des revendications précédentes, la composition thermoplastique contenant
a) au moins 50 à 99,999 % en poids dudit au moins un polymère thermoplastique,
b) 0,001 à 10 % en poids dudit au moins un lubrifiant et
c) 0 à 49,999 % en poids des autres additifs,
chaque fois par rapport au poids total de la composition thermoplastique, la somme des composants a) à c) étant égale à 100 % en poids.

14. Procédé pour la préparation d'une composition thermoplastique, par mise en contact d'au moins les composants suivants de la composition
A) au moins 50 à 99,999 % en poids, par rapport au poids total de la composition thermoplastique, d'au moins un polymère thermoplastique ;
B) 0,001 à 10 % en poids, par rapport au poids total de la composition thermoplastique, d'au moins un lubrifiant tel que défini dans les revendications 1 - 13 ; et
C) 0 à 49,999 % en poids, par rapport au poids total de la composition thermoplastique, d'au moins un additif
la somme des composants A) à C) étant égale à 100 % en poids.

15. Composition thermoplastique, pouvant être obtenue par le procédé selon la revendication 14.

16. Procédé pour la production d'un corps moulé, comportant les étapes de processus :
I) la disposition d'une composition thermoplastique selon l'une quelconque des revendications 1 à 13 ou 15.
II) le chauffage de la composition thermoplastique jusqu'à la température de transition vitreuse du polymère thermoplastique ou jusqu'à une température au-dessus de la température de transition vitreuse du polymère thermoplastique ;
III) la production d'un corps moulé à partir de la composition thermoplastique chauffée, préparée dans l'étape II) du procédé.

17. Procédé selon la revendication 16, dans lequel dans une autre étape IV) du procédé on réduit dans la section transversale de sa masse au moins une zone partielle du corps moulé obtenu dans l'étape III) du procédé, par rapport à l'étape III) du procédé.

18. Procédé selon la revendication 16 ou 17, dans lequel le corps moulé est choisi dans un groupe consistant en un récipient, un film, une fibre, un profilé et un tube.

19. Corps moulé, pouvant être obtenu par un procédé selon l'une quelconque des revendications 16 à 18.

20. Utilisation d'un mélange réactionnel qui peut être obtenu par la mise en réaction d'un composant comportant des triglycérides saturés avec un alcool primaire, en tant que lubrifiant dans des compositions thermoplastiques ;
le mélange réactionnel, qui outre le produit de transestérification (ester d'acide gras et d'alcool primaire), contenant également des esters partiels de glycérol, de l'alcool primaire n'ayant pas réagi et du triglycéride n'ayant pas réagi ; et
l'alcool primaire étant un polyol comportant plus de deux groupes OH.
